# EUROPEAN PATENT APPLICATION

(11) **EP 4 134 026 A1**
(43) Date of publication of application: **15.02.2023**
(21) Application number: 21808277.4
(22) Date of filing: 06.04.2021
(51) Int. Cl.: A61B 18/02

(54) **MONITORING DEVICE AND MONITORING METHOD THEREFOR**

(30) Priority: 19.05.2020 CN 202010424353
(71) Applicant: Microport Aesthetics Shanghai (Group) Co., Ltd., Shanghai 201203 (CN)
(72) Inventor: HUANG, Chubo, Jiaxing, Zhejiang 314051 (CN); ZHOU, Chuang, Jiaxing, Zhejiang 314051 (CN)
(74) Representative: Arnold & Siedsma
(86) International application number: PCT/CN2021/085615
(87) International publication number: WO 2021/232974

(57) **Abstract**

A monitoring equipment and a monitoring method thereof. An electrode pair (140) and a detection branch (150) are provided on a treatment device (100), when the treatment device (100) has been successfully adsorbed, that is, when an antifreeze film (130) is attached to a treatment surface (120), the antifreeze film (130) is electrically connected to the electrode pair (140), and the electrode pair (140) not only applies a stimulation to the detection branch (150), but also applies a stimulation to the antifreeze film (130). That is to say, the detection branch (150) is connected in parallel with the antifreeze film (130), and the detection branch (150) may detect a current of a loop. According to the current, a main control apparatus (200) may obtain a resistance value corresponding to the antifreeze film (130). According to a pre-stored resistance-temperature correspondence table, a temperature of the antifreeze film (130) may be obtained. Compared with a temperature detected directly in a traditional technology, the temperature obtained by detecting the resistance value has a higher accuracy, thereby enhancing safety of a treatment process.

## Description

### TECHNICAL FIELD

The present application relates to the field of medical device, and in particular, to a monitoring equipment and a monitoring method thereof.

### BACKGROUND

CoolSculpting technique is non-invasive, effective and selective, and may perform selective local fat reduction in the human body. Excessive freezing may cause damage to the human body, so it is necessary to monitor a state of the treatment device during a cryotherapy process to enhance safety of the treatment. The current method for monitoring the working state of the treatment device mainly determines the working state of the treatment device by detecting the magnitude and change rate of the temperature difference of a treatment surface of the treatment device. When the treatment device moves to a hotter area of the skin, or when the treatment device is no longer in contact with the skin, the temperature will change, so that the working state of the treatment device may be monitored by detecting the change magnitude and the change rate of the temperature. However, since the treatment device is provided with an antifreeze film, the accuracy of temperature detection will be affected, so that it is not sensitive enough to determine the movement of the treatment device by detecting the temperature difference of the treatment surface, and the skin temperature cannot be accurately indicated by detecting the temperature of the treatment surface.

### SUMMARY

Based on this, it is necessary to provide a monitoring equipment and a monitoring method thereof.

A monitoring equipment includes a treatment device and a main control device.

The treatment device includes a housing, a treatment surface and an antifreeze film, and the treatment surface is disposed between the housing and the antifreeze film.

The treatment device further includes a detection branch, the detection branch includes a current detection unit, and the current detection unit is configured to detect a current of the detection branch. The treatment device further includes an electrode pair, and the electrode pair is connected in parallel with the detection branch and then connected to a constant voltage source.

The main control device is electrically connected to the treatment device.

When the antifreeze film is attached to the treatment surface, the antifreeze film is connected to the constant voltage source through the electrode pair, the main control device is configured to receive the current detected by the current detection unit, and obtain a resistance value of the antifreeze film according to the branch current, and obtain a temperature of the antifreeze film according to the resistance value and a pre-stored resistance-temperature correspondence table.

In one of the embodiments, the electrode pair is disposed on the treatment surface and connected to the constant voltage source through a first connection wire embedded in the treatment surface, and a material of the treatment surface is an insulating material.

The resistance-temperature correspondence table includes a resistance-temperature correspondence table of the antifreeze film.

In one of the embodiments, the electrode pair is disposed on the antifreeze film and connected to the constant voltage source through a second connection wire embedded in the antifreeze film, the electrode pair is insulated from the antifreeze film by an insulating gasket disposed around the electrode pair.

The resistance-temperature correspondence table includes a relationship between the temperature and a total resistance value of the antifreeze film and the treatment surface.

In one of the embodiments, the electrode pair includes a first electrode and a second electrode.

The first electrode is disposed on the antifreeze film and connected to a first pole of the constant voltage source through a first connection wire embedded in the antifreeze film.

The second electrode is disposed on the treatment surface and connected to a second pole of the constant voltage source through a second connection line embedded in the treatment surface. A material of the treatment surface is an insulating material.

The resistance-temperature correspondence table includes a resistance-temperature correspondence table of the antifreeze film.

In one of the embodiments, the detection branch further includes a resistor R0, the resistor R0 is connected in series with the current detection unit, and the current detection unit is configured to detect the branch current I0 flowing through the resistor R0.

When the antifreeze film is attached to the treatment surface, the electrode pair forms a loop and is connected in parallel with the current detection unit and the resistor R0.

In one of the embodiments, the main control device includes a control unit and an operation unit connected to the control unit.

The control unit is configured to obtain the branch current I0 and a total current I1 of the constant voltage source.

The operation unit is configured to compare the branch current I0 and the total current I1, to determine that the antifreeze film is attached to the treatment surface when the branch current I0 is not equal to the total current I1, and to determine that the antifreeze film is not attached to the treatment surface when the branch current I0 is equal to the total current I1.

In one of the embodiments, the control unit is further configured to obtain a voltage of the constant voltage source.

The operation unit is further configured to, when the antifreeze film is attached to the treatment surface, calculate the resistance value of the antifreeze film according to the voltage and a difference between the total current I1 and the branch current I0.

In one of the embodiments, the main control device further includes a storage unit and an instruction generation unit, wherein the storage unit is configured to store the resistance-temperature correspondence table, and the instruction generation unit is configured to generate a control instruction including an alarm instruction.

The control unit is configured to obtain the temperature of the antifreeze film according to the resistance value of the antifreeze film and the resistance-temperature correspondence table.

The operation unit is further configured to compare the temperature of the antifreeze film and a preset temperature. The instruction generation unit is configured to, when the temperature of the antifreeze film is less than or equal to the preset temperature, generate the alarm instruction.

In one of the embodiments, the instruction generation unit is configured to generate the alarm instruction when the antifreeze film is not attached to the treatment surface.

In one of the embodiments, the monitoring equipment further includes an instruction execution module configured for executing an alarm according to the alarm instruction generated by the instruction generation unit.

The instruction execution module is arranged on the treatment device or on the main control device.

A monitoring method of the monitoring equipment above, including: detecting the branch current I0 of the detection branch, judging whether the antifreeze film is attached to the treatment surface according to the branch current 10, calculating the resistance value of the antifreeze film according to the branch current I0 when the antifreeze film is attached to the treatment surface, and obtaining the temperature of the antifreeze film according to the resistance value and the pre-stored resistance-temperature correspondence table.

In one of the embodiments, the monitoring method further includes: comparing the temperature of the antifreeze film and a preset temperature; and alarming and/or turning off the constant voltage source when the temperature of the antifreeze film is less than or equal to the preset temperature.

In one of the embodiments, the judging whether the antifreeze film is attached to the treatment surface according to the branch current I0 includes: obtaining a total current I1 of the constant voltage source; comparing the branch current I0 and the total current I1; and determining that the antifreeze film is attached to the treatment surface when the branch current I0 is not equal to the total current I1.

In one of the embodiments, the judging whether the antifreeze film is attached to the treatment surface according to the branch current I0 further includes: determining that the antifreeze film is not attached to the treatment surface when the branch current I0 is equal to the total current I1; and alarming and/or turning off the constant voltage source.

In one of the embodiments, the electrode pair is provided on the treatment surface and/or the antifreeze film.

In the monitoring equipment and the monitoring method thereof, the electrode pair and the detection branch are arranged on the treatment device. When the treatment device is successfully adsorbed, that is, the antifreeze film is attached to the treatment surface, the antifreeze film is electrically connected to the electrode pair, and the electrode pair not only applies an excitation to the detection branch, but also applies an excitation to the antifreeze film, that is to say, the detection branch is connected in parallel with the antifreeze film, and the detection branch may detect the current of the loop. The main control device may obtain the resistance value according to the current. The temperature of the antifreeze film may be obtained according to the pre-stored resistance-temperature correspondence table. Compared with the directly detected temperature in the traditional technology, the temperature obtained by detecting the resistance value in this embodiment has higher accuracy, thereby enhancing the safety of the treatment process.

### BRIEF DESCRIPTION OF THE DRAWINGS

FIG. 1 is a schematic view showing a monitoring equipment of an embodiment of the present application.
FIG. 2 is a schematic view showing the monitoring equipment of another embodiment of the present application.
FIG. 3 is a schematic view showing the monitoring equipment of yet another embodiment of the present application.
FIG. 4 is a schematic view showing the monitoring equipment of yet another embodiment of the present application.
FIG. 5 is a flowchart of a monitoring method of a monitoring equipment according to an embodiment of the present application.

### DETAILED DESCRIPTION OF THE EMBODIMENTS

In order to make the objectives, features and advantages of the present application clearer and to be better understood, the specific embodiments of the present application will be described in detail hereinafter with reference to the accompanying drawings. In the following description, numerous specific details are set forth to make the present application to be thoroughly understood. However, the present application may be implemented in other ways different from those described herein, and those skilled in the art may make similar improvements without departing from the connotation of the present application. Therefore, the present application is not limited by the specific embodiments disclosed hereinafter.

It should be noted that when an element is referred to as being "disposed on" another element, it may be directly on the other element or intervening elements may also be provided. When an element is referred to as being "connected to" another element, it may be directly connected to the other element or intervening elements may also be provided. The terms "vertical", "horizontal", "left", "right" and similar expressions used herein are for the purpose of illustration only but not represent the only embodiment.

Unless otherwise defined, all technical and scientific terms used herein have the same meaning as commonly understood by one of the ordinary skill in the technical field to which this application belongs. The terms used herein in the specification of the present application are for the purpose of describing specific embodiments only, but not intended to limit the present application.

As described in the background, in the traditional CoolSculpting technique, it is necessary to monitor the temperature of the treatment surface to monitor the change of the treatment state. Usually, the change of the treatment state is determined by directly detecting the temperature difference of the treatment surface during a period of time. However, due to the arrangement of the antifreeze film, the accuracy of a detection result of the directly detected temperature of the treatment surface is not high, and the directly detected temperature cannot accurately represent the skin temperature. The present application monitors the working state of the treatment device by monitoring a resistance value of the antifreeze film. Electrical conductivity is one of the properties of a substance, and the electrical conductivity has a great correlation with a temperature, therefore, the detection accuracy may be improved by obtaining the temperature of the antifreeze film based on a resistance-temperature correspondence table of the antifreeze film obtained in advance through experiments and the resistance value of the antifreeze film detected during the treatment.

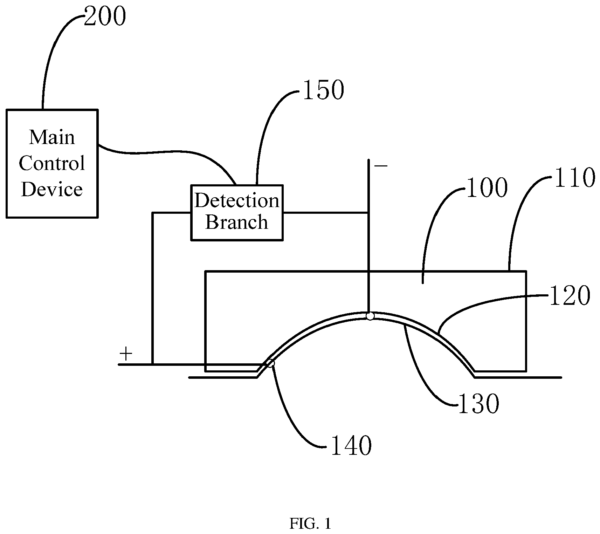

As shown in FIG. 1, an embodiment of the present application provides a monitoring equipment, including a treatment device 100 and a main control device 200. In this embodiment, in an embodiment, the main control device 200 may be a microprocessor or a microcontroller. The treatment device 100 is configured to be adsorbed on a treatment area to cool the treatment area to implement CoolSculpting. In an embodiment, the treatment device may be a handheld treatment device, for example, the treatment device 100 may be a vacuum suction handle. The treatment device 100 includes a housing 110, a treatment surface 120 and an antifreeze film 130. The treatment surface 120 is disposed between the housing 110 and the antifreeze film 130 to cool the treatment area. When the treatment device 100 is adsorbed on the treatment area, the antifreeze film 130 is attached to the treatment area to prevent the treatment area from frostbite.

The treatment device 100 further includes an electrode pair 140 and a detection branch 150. In an embodiment, the detection branch 150 may be a detection circuit. The detection branch 150 includes a current detection unit (not shown in FIG.1), and the current detection unit is configured to detect a current of the detection branch 150. In an embodiment, the current detection unit may be a current sensor. The treatment device 100 further includes the electrode pair 140, and the electrode pair 140 is connected in parallel with the detection branch 150 and then connected to a constant voltage source. The electrode pair 140 may include a first electrode 141 and a second electrode 142. The first electrode 141 is connected to the positive pole of the constant voltage source, and the second electrode 142 is connected to the negative pole of the constant voltage source. Alternatively, the first electrode 141 is connected to the negative pole of the constant voltage source, and the second electrode 142 is connected to the positive pole of the constant voltage source. The constant voltage source may be provided by the treatment device 100 or by the main control device 200. When the antifreeze film 130 is attached to the treatment surface 120, that is, the treatment device 100 is successfully adsorbed, the antifreeze film 130 is connected to the constant voltage source through the electrode pair 140, and the antifreeze film 130 is connected in parallel with the detection branch 150.

The main control device 200 is connected to the detection branch 150 to receive the current detected by the detection branch 150 and obtain a resistance value of the antifreeze film 130 according to the current. A resistance-temperature correspondence table is pre-stored in the main control device 200. Therefore, the main control device 200 may obtain the temperature of the antifreeze film 130 by looking up the resistance-temperature correspondence table according to the obtained resistance value.

In the monitoring equipment provided by the embodiment above, the electrode pair 140 and the detection branch 150 are disposed on the treatment device 100, and the electrode pair 140 is connected in parallel with the detection branch 150 and then connected to the constant voltage source. When the treatment device 100 is successfully adsorbed, that is, the antifreeze film 130 is attached to the treatment surface 120, the electrode pair 140 forms a loop, that is, the detection branch 150 is connected in parallel with the antifreeze film 130. The current detection unit may detect the current of the detection branch 150. The main control device 200 may obtain the resistance value of the antifreeze film 130 according to the current. The temperature of the antifreeze film 130 may be obtained according to the pre-stored resistance-temperature correspondence table. Compared with the directly detected temperature in the traditional technology, the temperature obtained by detecting the resistance value in this embodiment has higher accuracy, thereby enhancing the safety of the treatment process.

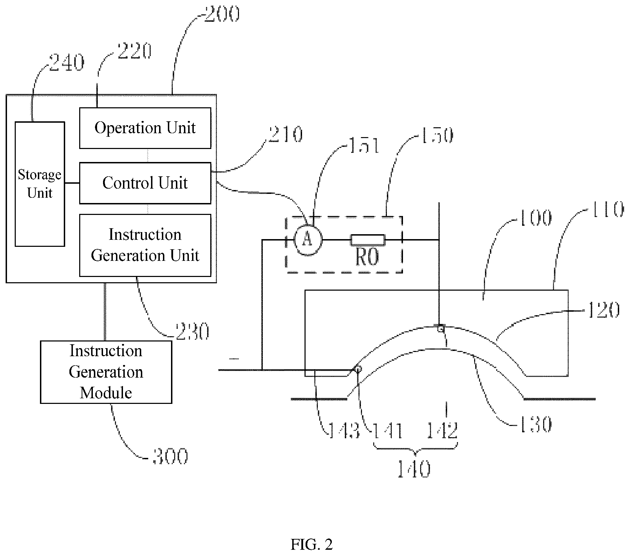

In an embodiment, as shown in FIG. 2, the electrode pair 140 is disposed on the treatment surface 120. The electrode pair 140 may be connected to the constant voltage source through the first connection wire 143 embedded in the treatment surface 120. The material of treatment surface 120 is an insulating material, so the treatment surface 120 will not conduct electricity when the electrode pair 140 is disposed on the treatment surface 120.

In this embodiment, the detection branch 150 further includes a resistor R0, the current detection unit 151 is connected in series with the resistor R0, and the resistor R0 is configured for voltage division. The first electrode 141 is connected to one end of the current detection unit 151 and a first pole of the constant voltage source, and the second electrode 142 is connected to one end of the resistor R0 and a second pole of the constant voltage source. The first pole of the constant voltage source is a positive pole, and the second pole of the constant voltage source is a negative pole. Alternatively, the first pole of the constant voltage source is the negative pole, and the second pole of the constant voltage source is the positive pole. The current detection unit 151 is configured to detect the branch current flowing through the resistor R0 and transmit it to the main control device 200. When the treatment surface 120 is attached to the antifreeze film 130, the antifreeze film 130 is in contact with the electrode pair 140. Since the material of the antifreeze film 130 is a conductive material, the constant voltage source also applies excitation to the antifreeze film 130 through the electrode pair 140, and the constant voltage source also applies excitation to the resistor R0, and in this case, the antifreeze film 130 is connected in parallel with the resistor R0. The current detection unit 151 may detect the branch current I0 flowing through the resistor R0, and the main control device 200 may obtain a total current I1 of the constant voltage source, so a current flowing through the antifreeze film 130 is I1-I0. The main control device 200 may also obtain a total voltage V1 of the constant voltage source, and obtain the resistance value of the antifreeze film 130 according to the total voltage V1 and the current flowing through the antifreeze film 130. The temperature of the antifreeze film 130 may be obtained according to the pre-stored resistance-temperature correspondence table of the antifreeze film 130.

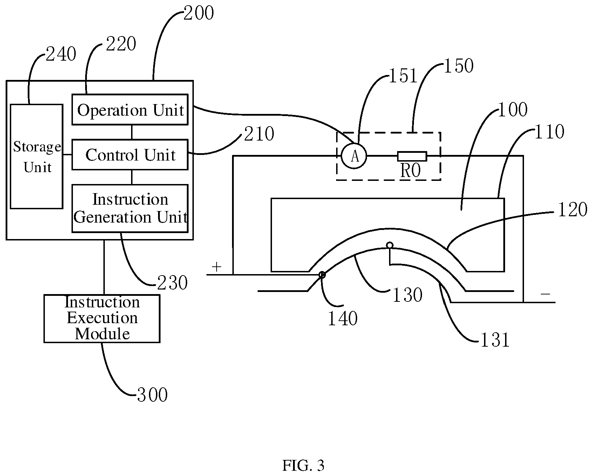

In another embodiment, as shown in FIG. 3, the electrode pair 140 may be disposed on the antifreeze film 130 and connected to the constant voltage source through the second connection wire 131 embedded in the antifreeze film 130. The second connection wire 131 is insulated from the antifreeze film 130. The electrode pair 140 is insulated from the antifreeze film 130 by an insulating gasket disposed around the electrode pair 140.

In this embodiment, the detection branch 150 includes the current detection unit 151 and the resistor R0. The current detection unit 151 is connected in series with the resistor R0, and the resistor R0 is configured for voltage division. The materials of the treatment surface 120 and the antifreeze film 130 are both conductive materials. When the treatment surface 120 is attached to the antifreeze film 130, the treatment surface 120 and the antifreeze film 130 may be equivalent to a film layer. When the treatment surface 120 is in contact with the electrode pair 140, the constant voltage source supplies power to the treatment surface 120 and the antifreeze film 130 through the electrode pair 140. If the first electrode 141 is connected to the positive pole of the constant voltage source, and if the second electrode 142 is connected to the negative pole of the constant voltage source, then the current in one branch flows through the first electrode 141, the treatment surface 120 and the antifreeze film 130, and flows to the second electrode 142, while the current in another branch flows through the positive pole of the constant voltage source, the current detection unit 151 and the resistor R0, and flows to the negative pole of the constant voltage source. An equivalent circuit is formed by the treatment surface 120 and the antifreeze film 130 which are connected in series and then connected in parallel with the resistor R0. The current detection unit 151 may detect the branch current I0 flowing through the resistor R0, and the main control device 200 may obtain the total current I1 and the total voltage V1 of the constant voltage source, so a total resistance value of the treatment surface 120 and the antifreeze film 130 is V1/(I1-I0). In this embodiment, the resistance-temperature correspondence table pre-stored in the main control device 200 is the corresponding relationship between the temperature and the total resistance value of the treatment surface 120 and the antifreeze film 130. Therefore, the temperature of the treatment surface 120 and the antifreeze film 130 may be obtained according to the total resistance value of the treatment surface 120 and the antifreeze film 130.

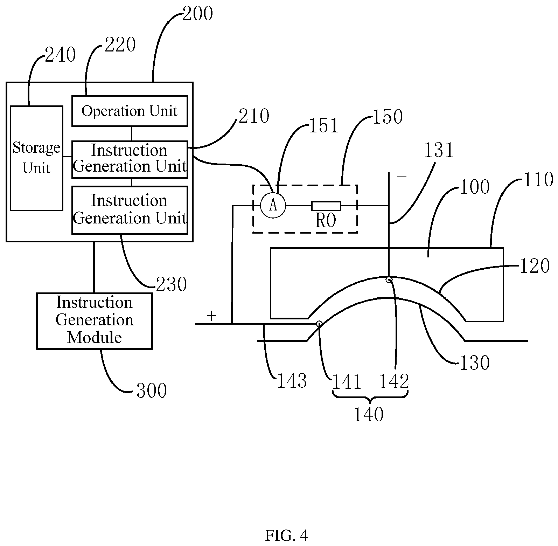

In yet another embodiment, as shown in FIG. 4, the electrode pair 140 includes the first electrode 141 and the second electrode 142. The first electrode 141 is disposed on the antifreeze film 130 and is connected to the first pole of the constant voltage source through a first connection wire 143 embedded in the treatment surface 120. The second electrode 142 is disposed on the treatment surface 120 and is connected to the second pole of the constant voltage source through the second connection wire 131 disposed on the treatment surface 120. The material of the treatment surface 120 is an insulating material.

In this embodiment, the detection branch 150 includes the current detection unit 151 and the resistor R0. The current detection unit 151 is connected in series with the resistor R0, and the resistor R0 is configured for voltage division. The electrode pair 140 is connected in parallel with the detection branch 150 and then connected to the constant voltage source. The first electrode 141 is connected to the first pole of the constant voltage source, and the second electrode 142 is connected to the second pole of the constant voltage source. As shown in FIG. 4, the first pole of the constant voltage source is the positive pole and the second pole of the constant voltage source is the negative pole. Alternatively, the first pole of the constant voltage source is the negative pole, and the second pole of the constant voltage source is the positive pole. The first electrode 141 is disposed on the antifreeze film 130, and the second electrode 142 is disposed on the treatment surface 120. When a vacuum adsorption is unsuccessful, that is, when the antifreeze film 130 is not attached to the treatment surface 120, and when the antifreeze film 130 is not connected to the constant voltage source, the current detected by the current detection unit is the branch current I0 flowing through the resistor R0. When the vacuum adsorption is successful, the antifreeze film 130 is attached to the treatment surface 120, and further, the excitation applied by the constant voltage source may flow through the positive pole of the constant voltage source, the first electrode 141 and the antifreeze film 130, then flows from the second electrode 142 to the negative pole of the constant voltage source, forming a current loop. The current loop is connected in parallel with the detection branch 150. The main control device 200 obtains the branch current I0 of the detection branch 150 by means of the current detection unit 151, and may also obtain the total voltage V1 and the total current I1 of the constant voltage source, thus obtaining the resistance value of the antifreeze film 130 according to the voltage V1 and the current (I1-I0) flowing through the antifreeze film 130. The main control device 20 may obtain the temperature of the antifreeze film 130 according to the pre-stored resistance-temperature correspondence table of the antifreeze film 130.

Further, in one of the embodiments, the main control device 200 includes a control unit 210, and an operation unit 220 and an instruction generation unit 230 and a storage unit 240 which are connected to the control unit. In an embodiment, the control unit 210 may be a microcontroller unit, the operation unit 220 may be an operator, and the instruction generation unit 230 may be an instruction generator, and the storage unit 240 may be a memory. The control unit 210 is connected to the current detection unit 151 to obtain a detected current I0 of the resistor R0. The control unit 210 is further configured to obtain the voltage V1 and the total current I1 of the constant voltage source. The operation unit 220 first compares the current I0 of the resistor R0 and the total current I1. When the current I0 of the resistance R0 is equal to the total current I1, it indicates that the antifreeze film 130 is not connected to the circuit, that is, the antifreeze film 130 is not attached to the treatment surface 120. When the current I0 of the resistance R0 is not equal to the total current I1, it indicates that the antifreeze film 130 is connected to the circuit, that is, the antifreeze film 130 is attached to the treatment surface 120.

When the antifreeze film 130 is not attached to the treatment surface 120, it indicates that the treatment handle falls off, then the instruction generation unit 230 generates control instructions, such as an alarm instruction, a power-off instruction, and the like. The monitoring equipment further includes an instruction execution module 300 configured for receiving the control instructions and executing corresponding actions, such as alarming, turning off the power source, and the like. In an embodiment, the instruction execution module 300 may be an audible and visual alarm. The instruction execution module 300 may include an alarm unit and a power button, and may be arranged on the treatment device 100 or on the main control device 200.

When the antifreeze film 130 is attached to the treatment surface, the operation unit obtains the resistance value of the antifreeze film 130 according to the current I0 of the resistance R0, the total current I1 and the voltage V1. The storage unit 240 stores a resistance-temperature correspondence table, and the control unit 210 may obtain the temperature of the antifreeze film 130 by looking up the resistance-temperature correspondence table according to the resistance value of the antifreeze film 130.

The storage unit 240 also stores a preset temperature T0. The operation unit 220 is configured to compare the obtained temperature of the antifreeze film 130 and the preset temperature T0. When the temperature of the antifreeze film 130 is less than or equal to the preset temperature T0, the temperature is too low and may cause damage to the treatment area, then the instruction generation unit 230 generates the alarm instruction and the power-off instruction. When the temperature of the antifreeze film 130 is greater than the preset temperature T0, the treatment continues, and the temperature of the antifreeze film 130 is detected in real time and calculated, till the temperature of the antifreeze film 130 is less than or equal to the preset temperature or till the treatment ends.

In the above embodiments, one electrode pair 140 is provided. Of course, the number of electrode pairs 140 may also be multiple. When the number of the electrode pairs 140 is multiple, the number of detection branches 150 is the same as the number of the electrode pairs 140, that is, one detection branch 150 is connected in parallel with one electrode pair 140, and the multiple electrode pairs may be arranged at different positions on the antifreeze film 130 and/or the treatment surface 120, respectively. The control unit 210 may obtain multiple resistance values, and an average value of the antifreeze film 130 is obtained to obtain the temperature of the antifreeze film 130.

Further, the electrode pair 140 in the above embodiments may also apply an electrical stimulation to the treatment area to enhance the treatment effect.

A monitoring method for a monitoring equipment of the present application is provided.

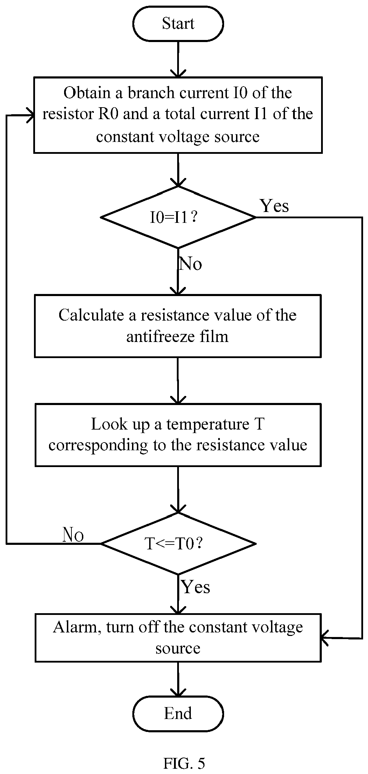

This embodiment is described by taking the electrode pair 140 installed on the treatment surface 120 as an example. As shown in FIG. 5, after the treatment device 100 is powered on, first, the current detection unit 151 detects the current I0 of the resistor R0, and transmits the current I0 to the control unit 210. The control unit 210 obtains the voltage V1 and the total current I1 of the constant voltage source. The operation unit 220 compares the current I0 of the resistance R0 and the total current I1. If the current I0 of the resistance R0 is equal to the total current I1, it indicates that the antifreeze film 130 is not attached to the treatment surface 120, that is, the treatment handle falls off, then the instruction generation unit 230 generates control instructions, such as an alarm command, a power-off command, and the like. The instruction execution module 300 receives the control instructions and executes corresponding actions according to the content of the control instructions.

If the current I0 of the resistance R0 is not equal to the total current I1, it indicates that the antifreeze film 130 is in contact with the treatment surface 120, and the resistance value of the antifreeze film 130 may be obtained. Specifically, the operation unit 220 calculates the resistance value of the antifreeze film 130 according to the current I0 of the resistor R0, the total current I1 and the voltage V1, and the resistance value of the antifreeze film 130 is V1/(I1-I0). The control unit 210 looks up the resistance-temperature correspondence table according to the resistance value of the antifreeze film 130 to obtain a current temperature T of the antifreeze film 130. The operation unit 220 is also configured to compare the current temperature T of the antifreeze film 130 and the preset temperature T0. When the temperature T of the antifreeze film 130 is less than or equal to the preset temperature T0, it indicates that the temperature of the antifreeze film is too low and may cause damage to the treatment area, then the instruction generation unit 230 generates the control instructions, such as the alarm instruction, the power-off instruction, and the like. The instruction execution module 300 receives the control instructions and executes the corresponding actions according to the content of the control instructions. When the temperature T of the antifreeze film 130 is greater than the preset temperature T0, the monitoring equipment continues to monitor till the treatment handle falls off, or till the temperature of the antifreeze film 130 is less than or equal to the preset temperature T0, or till the treatment ends.

Various technical features of the embodiments above may be combined arbitrarily. For the sake of brevity, not all possible combinations of the technical features in the embodiments above are described. However, as long as there is no contradiction between the combinations of these technical features, all combinations should be regarded as the scope described in this specification.

The embodiments above only represent several embodiments of the present application, and the descriptions thereof are relatively specific and detailed, but should not be construed as a limitation on the scope of the patent application. It should be noted that, for those skilled in the art, various modifications and improvements may be made without departing from the concept of the present application, and all the modifications and improvements belong to the protection scope of the present application. Therefore, the protection scope of the present patent application shall be subject to the appended claims.

## Claims

1. A monitoring equipment, **characterized by** comprising: a treatment device and a main control device, wherein:
the treatment device comprises a housing, a treatment surface and an antifreeze film, and the treatment surface is disposed between the housing and the antifreeze film;
the treatment device further comprises a detection branch, the detection branch comprises a current detection unit, and the current detection unit is configured to detect a current of the detection branch;
the treatment device further comprises an electrode pair, and the electrode pair is connected in parallel with the detection branch and then connected to a constant voltage source;
the main control device is electrically connected to the treatment device; and
when the antifreeze film is attached to the treatment surface, the antifreeze film is connected to the constant voltage source through the electrode pair, the main control device is configured to receive the current detected by the current detection unit, and obtain a resistance value of the antifreeze film according to the current, and obtain a temperature of the antifreeze film according to the resistance value and a pre-stored resistance-temperature correspondence table.

2. The monitoring equipment of claim 1, wherein:
the electrode pair is disposed on the treatment surface and connected to the constant voltage source through a first connection wire embedded in the treatment surface, and a material of the treatment surface is an insulating material; and
the resistance-temperature correspondence table comprises a resistance-temperature correspondence table of the antifreeze film.

3. The monitoring equipment of claim 1, wherein:
the electrode pair is disposed on the antifreeze film and connected to the constant voltage source through a second connection wire embedded in the antifreeze film, the electrode pair is insulated from the antifreeze film by an insulating gasket disposed around the electrode pair; and
the resistance-temperature correspondence table comprises a relationship between the temperature and a total resistance value of the antifreeze film and the treatment surface.

4. The monitoring equipment of claim 1, wherein:
the electrode pair comprises a first electrode and a second electrode;
the first electrode is disposed on the antifreeze film and connected to a first pole of the constant voltage source through a first connection wire embedded in the antifreeze film;
the second electrode is disposed on the treatment surface and connected to a second pole of the constant voltage source through a second connection wire embedded in the treatment surface;
a material of the treatment surface is an insulating material; and
the resistance-temperature correspondence table comprises a resistance-temperature correspondence table of the antifreeze film.

5. The monitoring equipment of any one of claims 2-4, wherein:
the detection branch further comprises a resistor R0, the resistor R0 is connected in series with the current detection unit, and the current detection unit is configured to detect the branch current I0 flowing through the resistor R0; and
when the antifreeze film is attached to the treatment surface, the electrode pair forms a loop and is connected in parallel with the current detection unit and the resistor R0.

6. The monitoring equipment of claim 5, wherein:
the main control device comprises a control unit and an operation unit connected to the control unit;
the control unit is configured to obtain the branch current I0 and a total current I1 of the constant voltage source;
the operation unit is configured to compare the branch current I0 and the total current I1, to determine that the antifreeze film is attached to the treatment surface when the branch current I0 is not equal to the total current I1, and to determine that the antifreeze film is not attached to the treatment surface when the branch current I0 is equal to the total current I1.

7. The monitoring equipment of claim 6, wherein:
the control unit is further configured to obtain a voltage of the constant voltage source; and
the operation unit is further configured to, when the antifreeze film is attached to the treatment surface, calculate the resistance value of the antifreeze film according to the voltage and a difference between the total current I1 and the branch current I0.

8. The monitoring equipment of claim 7, wherein:
the main control device further comprises a storage unit and an instruction generation unit, wherein the storage unit is configured to store the resistance-temperature correspondence table, and the instruction generation unit is configured to generate a control instruction comprising an alarm instruction;
the control unit is configured to obtain the temperature of the antifreeze film according to the resistance value of the antifreeze film and the resistance-temperature correspondence table;
the operation unit is further configured to compare the temperature of the antifreeze film and a preset temperature; and
the instruction generation unit is configured to, when the temperature of the antifreeze film is less than or equal to the preset temperature, generate the alarm instruction.

9. The monitoring equipment of claim 8, wherein the instruction generation unit is configured to generate the alarm instruction when the antifreeze film is not attached to the treatment surface.

10. The monitoring equipment of claim 8 or 9, wherein
the monitoring equipment further comprises an instruction execution module configured for executing an alarm according to the alarm instruction generated by the instruction generation unit; and
the instruction execution module is arranged on the treatment device or on the main control device.

11. The monitoring equipment of claim 1, wherein:
the treatment device comprises multiple electrode pairs and multiple detection branches; one of the multiple detection branches is connected in parallel with one of the multiple electrode pairs and then connected to the constant voltage source;
the main control device is configured to: receive detected multiple currents of the multiple detection branches; obtain multiple resistance values according to the multiple currents; take an average value of the multiple resistance values as the resistance value of the antifreeze film; and obtain the temperature of the antifreeze film according to the resistance value of the antifreeze film and the pre-stored resistance-temperature correspondence table.

12. The monitoring equipment of claim 11, wherein the multiple electrode pairs are respectively disposed at different positions of the antifreeze film and/or the treatment surface.

13. The monitoring equipment of claim 1, wherein the electrode pair is capable of applying an electrical stimulation to a treatment area when the electrode pair is disposed on the treatment surface.

14. A monitoring method of the monitoring equipment of claim 1, **characterized by** comprising:
detecting the branch current I0 of the detection branch;
judging whether the antifreeze film is attached to the treatment surface according to the branch current 10;
calculating the resistance value of the antifreeze film according to the branch current I0 when the antifreeze film is attached to the treatment surface; and
obtaining the temperature of the antifreeze film according to the resistance value and the pre-stored resistance-temperature correspondence table.

15. The monitoring method of claim 14, further comprising:
comparing the temperature of the antifreeze film and a preset temperature; and
alarming and/or turning off the constant voltage source when the temperature of the antifreeze film is less than or equal to the preset temperature.

16. The monitoring method of claim 14, wherein, the judging whether the antifreeze film is attached to the treatment surface according to the branch current I0 comprises:
obtaining a total current I1 of the constant voltage source;
comparing the branch current I0 and the total current I1; and
determining that the antifreeze film is attached to the treatment surface when the branch current I0 is not equal to the total current I1.

17. The monitoring method of claim 16, wherein, the judging whether the antifreeze film is attached to the treatment surface according to the branch current I0 further comprises:
determining that the antifreeze film is not attached to the treatment surface when the branch current I0 is equal to the total current I1; and
alarming and/or turning off the constant voltage source.

18. The monitoring method of claim 14, wherein the electrode pair is provided on the treatment surface and/or the antifreeze film.
